# EUROPÄISCHE PATENTANMELDUNG

(11) **EP 3 725 760 A1**
(43) Veröffentlichungstag der Anmeldung: **21.10.2020**
(21) Anmeldenummer: 19180879.9
(22) Anmeldetag: 18.06.2019
(51) Int. Cl.: C07C 29/15, C07C 29/151, C01B 3/00, C07C 31/04

(54) **VERFAHREN UND ANLAGE ZUR SYNTHESE VON METHANOL**

(30) Priorität: 18.04.2019 DE 102019110392
(71) Anmelder: thyssenkrupp Industrial Solutions AG, 45143 Essen (DE); GasConTec GmbH, 61348 Bad Homburg v. d. Höhe (DE)
(72) Erfinder: Schulz, Alexander, 60439 Frankfurt (DE); Banik, Beata, 45731 Waltrop (DE)
(74) Vertreter: Patentanwälte Bauer Vorberg Kayser

(57) **Zusammenfassung**

Die Erfindung betrifft ein Verfahren zur Synthese von Methanol (1), wobei ein kohlenstoffhaltiger Energieträgerstrom (11) einer Synthesegasreaktoranordnung (13) zum Gewinnen eines Synthesegasstroms (2) mit Wasserstoff und Kohlenstoffoxiden zugeführt wird, wobei der Synthesegasstrom (2) einer ersten Reaktorstufe (21a) einer Methanol-Reaktoranordnung (4) zur teilweisen Umwandlung in Methanol (1) zugeführt wird, wobei der Synthesegasstrom (2) in der Synthesegasreaktoranordnung (13) mit einem Erzeugungsdruck gewonnen wird, welcher höher ist als der Synthesedruck, mit welchem der Synthesegasstrom (2) in der ersten Reaktorstufe (21a) teilweise in Methanol (1) umgewandelt wird, wobei aus der Methanol-Reaktoranordnung (4) ein Restgasstrom (15) mit unreagierten Kohlenstoffoxiden gewonnen wird, welcher Restgasstrom (15) einem Recyclekompressor (14) zur Druckerhöhung des Restgasstroms (15) zugeführt wird, wobei der druckerhöhte Restgasstrom (15) der Methanol-Reaktoranordnung (4) zur teilweisen Umwandlung in Methanol (1) zugeführt wird, wobei vor Zuführung zu der ersten Reaktorstufe (21a) der Synthesegasstrom (2) einer Wärmerückgewinnungsvorrichtung (10) zum Rückgewinnen von Wärme aus dem Synthesegasstrom (2) zugeführt wird, wobei ein Rückgewinnungsstrom (6) mit unreagiertem Wasserstoff aus einem unreagierten Restgas (16a) der ersten Reaktorstufe (21a) einer Wasserstoffrückgewinnungsanordnung (5) zum Gewinnen eines H-Recyclestrom (7) mit dem unreagierten Wasserstoff des Rückgewinnungsstroms (6) zugeführt wird, welcher unreagierte Wasserstoff des Rückgewinnungsstroms (6) erneut der ersten Reaktorstufe (21a) zur zumindest teilweisen Umwandlung in Methanol (1) zugeführt wird. Das Verfahren ist dadurch gekennzeichnet, dass der unreagierte Wasserstoff des Rückgewinnungsstroms (6) von der ersten Reaktorstufe (21a) bis zur erneuten Zuführung zur ersten Reaktorstufe (21a) durch den Recyclekompressor (14) mit den unreagierten Kohlenstoffoxiden genau einmal druckerhöht wird. Ebenso betrifft die Erfindung eine entsprechende Anlage zur Synthese von Methanol (1).

## Beschreibung

Die Erfindung betrifft ein Verfahren zur Synthese von Methanol gemäß dem Oberbegriff von Anspruch 1 sowie eine Anlage zur Synthese von Methanol gemäß dem Oberbegriff von Anspruch 15.

Die Herstellung von Methanol findet regelmäßig in einem Reaktor einer Anlage für die Synthese von Methanol statt, welchem Reaktor ein Synthesegasstrom mit Wasserstoff und Kohlenstoffoxiden zugeführt wird und in welchem die exotherme Reaktion zur Herstellung von Methanol abläuft.

Grundsätzlich ist es zweckmäßig, die Methanolsynthese bei einem hohen Druck durchzuführen. Daher ist regelmäßig in der Anlage ein Synthesegaskompressor vorgesehen, welcher den Synthesegasstrom auf den gewünschten Druck komprimiert. Allerdings ist ein solcher Synthesegaskompressor insbesondere energetisch besonders ressourcenintensiv und daher ein wesentlicher Kostenfaktor bei der Synthese von Methanol.

Im Stand der Technik gibt es Ansätze, auf einen solchen Synthesegaskompressor zu verzichten. Das amerikanische Patent US 6,881,758 B2 beschreibt eine Anlage zur Synthese von Methanol, welche ohne einen Synthesegaskompressor auskommt. Speziell wird das Synthesegas in dem entsprechenden Reaktor unter Zusatz von druckerhöhtem Sauerstoff beispielsweise durch autotherme Reformierung bereits mit einem Druck von 60 bar bereitgestellt, sodass das Synthesegas ohne Druckerhöhung dem Methanolsynthesereaktor zugeführt werden kann.

Nachteilig an diesem Stand der Technik ist, dass beim Erzeugen des Synthesegases durch autotherme Reformierung die für die Methanolsynthese bevorzugte Stöchiometrie nicht erreicht werden kann.

Die Offenlegungsschrift WO 2005/108336 A1 aus dem Stand der Technik beschreibt ebenfalls eine Anlage zur Synthese von Methanol, bei welcher ebenfalls kein Synthesegaskompressor vorgesehen ist. Das Synthesegas wird bei der in der WO 2005/108336 A1 gezeigten Anlage durch nicht-katalytische partielle Oxidation gewonnen. Zwar findet eine Rückführung von Wasserstoff, welcher durch eine PSA aus einem Restgas des Methanolreaktors gewonnen wurde, zu diesem Methanolreaktor statt, doch wird dieser Wasserstoff entweder überhaupt nicht komprimiert oder es findet eine Komprimierung mit einem eigenen Kompressor statt. Jedoch ist einerseits die Komprimierung eines reinen Wasserstoffstroms aufwendig und andererseits das Vorsehen eines weiteren Kompressors kostspielig.

Die EP 2 011 564 A1 aus dem Stand der Technik, von welcher die vorliegende Erfindung ausgeht, sieht sowohl den Verzicht auf einen Synthesegaskompressor als auch die Maßnahme vor, dass ein Wasserstoffstrom, welcher mittels einer PSA aus unreagiertem Restgas gewonnen wurde, wieder dem Recyclekompressor zugeführt wird. Nachteilig an der hier gezeigten Lösung ist allerdings, dass der der PSA zugeführte Strom bereits durch den Recyclekompressor eine Druckerhöhung erfahren hat. Dadurch, dass die Rückführung vom Ausgang des Recyclekompressors zum Eingang des Recyclekompressors und damit in einem eigenen Kreislauf erfolgt, ist eine Dimensionierung des Recyclekompressors erforderlich, welche größer ist, als an sich nötig wäre.

Ausgehend von diesem Stand der Technik besteht die Aufgabe der Erfindung daher darin, das aus dem Stand der Technik bekannte Verfahren zur Synthese von Methanol ohne Synthesegaskompressor und die aus dem Stand der Technik bekannte Anlage zur Synthese von Methanol ohne Synthesegaskompressor dahingehend zu verbessern und weiterzuentwickeln, dass weiterhin kein eigener Kompressor für den zurückzuführenden Wasserstoff erforderlich ist und der Recyclekompressor kleiner dimensioniert werden kann.

Bezogen auf ein Verfahren zur Synthese von Methanol gemäß dem Oberbegriff von Anspruch 1 wird diese Aufgabe durch die Merkmale des kennzeichnenden Teils von Anspruch 1 gelöst. Bezogen auf eine Anlage zur Synthese von Methanol gemäß dem Oberbegriff von Anspruch 15 wird diese Aufgabe durch die Merkmale des kennzeichnenden Teils von Anspruch 15 gelöst.

Der Erfindung liegt die Erkenntnis zugrunde, dass der extrahierte Wasserstoff aus der Wasserstoffrückgewinnung so geführt werden kann, dass er zwischen dem Austritt aus der ersten Reaktorstufe der Methanolsynthese und der erneuten Zuführung zu der ersten Reaktorstufe der Methanolsynthese genau einmal eine Druckerhöhung durch den Recyclekompressor erfährt. Auf diese Weise wird verhindert, dass eine wiederholte und damit im Grunde überflüssige Druckerhöhung des Wasserstoffs erfolgt. Dadurch kann dann die Dimensionierung des Recyclekompressors verringert werden.

Das vorschlagsgemäße Verfahren dient der Synthese von Methanol. Bei dem vorschlagsgemäßen Verfahren wird ein kohlenstoffhaltiger Energieträgerstrom einer Synthesegasreaktoranordnung zum Gewinnen eines Synthesegasstroms mit Wasserstoff und Kohlenstoffoxiden zugeführt. Der Synthesegasstrom weist also Wasserstoff, Kohlenstoffmonoxid und Kohlenstoffdioxid auf und kann daneben noch weitere Bestandteile wie insbesondere Stickstoff und Edelgase aufweisen. Der Synthesegasstrom kann auch als Frischgasstrom bezeichnet werden.

Ebenso wird bei dem vorschlagsgemäßen Verfahren der Synthesegasstrom einer ersten Reaktorstufe einer Methanol-Reaktoranordnung zur teilweisen Umwandlung in Methanol zugeführt. Das Merkmal der teilweisen Umwandlung in Methanol begründet sich darin, dass ein nicht umgesetzter Rest an Edukten aus der Methanol-Reaktoranordnung austritt und daher die Umwandlung nicht vollständig abläuft. Die Methanol-Reaktoranordnung kann mehrere Reaktorstufen oder nur eine einzelne Reaktorstufe aufweisen. Weist die Methanol-Reaktoranordnung nur eine einzelne Reaktorstufe auf, so handelt es sich bei der ersten Reaktorstufe um diese einzige Reaktorstufe der Methanol-Reaktoranordnung. Die erste Reaktorstufe der Methanol-Reaktoranordnung ist diejenige Reaktorstufe der Methanol-Reaktoranordnung, welcher der Synthesegasstrom zugeführt wird, bevor er oder ein verbliebener Restgasstrom einer weiteren Reaktorstufe zugeführt wird. Die erste Reaktorstufe ist insoweit die prozesstechnisch zuerst gelagerte Reaktorstufe der Methanol-Reaktoranordnung. Dieser Umstand deckt sich mit der möglichen Bezeichnung des Synthesegasstroms als Frischgasstrom. Jede einzelne Reaktorstufe der Methanol-Reaktoranordnung kann dabei mehrere, prozesstechnisch zueinander parallel geschaltete Einzelreaktoren für die Methanolsynthese aufweisen.

Gemäß dem vorschlagsgemäßen Verfahren wird der Synthesegasstrom in der Synthesegasreaktoranordnung mit einem Erzeugungsdruck gewonnen, welcher höher ist als der Synthesedruck, mit welchem der Synthesegasstrom in der ersten Reaktorstufe teilweise in Methanol umgewandelt wird. Mit anderen Worten erfährt der Synthesegasstrom - und damit das Synthesegas - ab Erzeugung im Ergebnis keine Druckerhöhung bis zum Erreichen der Methanol-Reaktoranordnung für die Methanolsynthese. Insbesondere erfolgt keine Druckerhöhung des Synthesegasstroms nach seiner Erzeugung durch einen der Methanolsynthese prozesstechnisch vorgelagerten - und damit der Synthesegasherstellung nachgelagerten - Kompressor. Ebenso kann es sein, dass nach Durchlaufen der Methanol-Reaktoranordnung verbliebenes Restgas eine Druckerhöhung erfährt. Dies wird untenstehend noch erläutert.

Beim vorschlagsgemäßen Verfahren ist vorgesehen, dass aus der Methanol-Reaktoranordnung ein Restgasstrom mit unreagierten Kohlenstoffoxiden gewonnen wird, welcher Restgasstrom einem Recyclekompressor zur Druckerhöhung des Restgasstroms zugeführt wird. Der Restgasstrom weist ebenso unreagierten Wasserstoff auf. Sollte die Methanol-Reaktoranordnung mehr als eine Reaktorstufe aufweisen, so kann dieser Restgasstrom nach einer beliebigen Reaktorstufe gewonnen werden.

Das vorschlagsgemäße Verfahren sieht weiter vor, dass der druckerhöhte Restgasstrom der Methanol-Reaktoranordnung zur teilweisen Umwandlung in Methanol zugeführt wird. Es handelt sich also um eine Rückführung des nun druckerhöhten Restgasstroms zur Methanol-Reaktoranordnung, aus welcher der Restgasstrom ja gewonnen wurde.

Das vorschlagsgemäße Verfahren sieht ebenso vor, dass vor Zuführung zu der ersten Reaktorstufe der Synthesegasstrom einer Wärmerückgewinnungsvorrichtung zum Rückgewinnen von Wärme aus dem Synthesegasstrom zugeführt wird. Diese Wärmerückgewinnungsvorrichtung ist mit anderen Worten prozesstechnisch zwischen der Synthesegasreaktoranordnung und der Methanol-Reaktoranordnung angeordnet. Hierbei ist zu beachten, dass die Wärmerückgewinnungsvorrichtung regelmäßig nur eine Stufe einer Wärmerückgewinnungsanordnung mit mehreren Wärmerückgewinnungsvorrichtungen darstellt. Anders ausgedrückt kann es sein, dass der Synthesegasstrom nur einer Wärmerückgewinnungsvorrichtung von mehreren, miteinander zusammenhängenden Wärmerückgewinnungsvorrichtungen vor Zuführung zu der Methanol-Reaktoranordnung zugeführt wird.

Das vorschlagsgemäße Verfahren sieht zusätzlich vor, dass ein Rückgewinnungsstrom mit unreagiertem Wasserstoff aus einem unreagierten Restgas der ersten Reaktorstufe einer Wasserstoffrückgewinnungsanordnung zum Gewinnen eines H-Recyclestroms mit dem unreagierten Wasserstoff des Rückgewinnungsstroms zugeführt wird. Es ergibt sich, dass der Wasserstoff des H-Recyclestroms zumindest teilweise und vorzugsweise vollständig dem unreagierten Wasserstoff des Rückgewinnungsstroms entspricht. Bei dem unreagierten Restgas kann es sein, dass es sich nur um einen Teil des gesamten unreagierten Gases aus der ersten Reaktorstufe handeln. Ebenso kann es sein, dass der unreagierte Wasserstoff nur ein Teil des gesamten unreagierten Wasserstoffs der ersten Reaktorstufe ist.

Das vorschlagsgemäße Verfahren sieht vor, dass dieser unreagierte Wasserstoff des Rückgewinnungsstroms erneut der ersten Reaktorstufe zur zumindest teilweisen Umwandlung in Methanol zugeführt wird. Die erneute Zuführung des unreagierten Wasserstoffes des Rückgewinnungsstroms zur ersten Reaktorstufe kann dabei sowohl direkt als auch indirekt erfolgen. In dem Fall der indirekten Zuführung wird der unreagierte Wasserstoff also zunächst anderen Vorrichtungen zugeführt.

Das vorschlagsgemäße Verfahren ist dadurch gekennzeichnet, dass der unreagierte Wasserstoff des Rückgewinnungsstroms von der ersten Reaktorstufe bis zur erneuten Zuführung zur ersten Reaktorstufe durch den Recyclekompressor mit den unreagierten Kohlenstoffoxiden genau einmal druckerhöht wird. Anders ausgedrückt findet zwischen dem Austritt des unreagierten Wasserstoffes aus der ersten Reaktorstufe und der erneuten Zuführung dieses unreagierten Wasserstoffes zu der ersten Reaktorstufe eine Druckerhöhung nur einmal statt, und zwar speziell durch den Recyclekompressor. Da der Recyclekompressor - wie bereits festgestellt - den Restgasstrom mit unreagierten Kohlenstoffoxiden druckerhöht, findet also diese Druckerhöhung durch den Recyclekompressor mit den unreagierten Kohlenstoffoxiden statt. Ebenso wird unreagierter Wasserstoff der ersten Reaktorstufe, welcher nicht der Wasserstoffrückgewinnungsanordnung zugeführt wurde, durch den Recyclekompressor druckerhöht.

An sich überflüssige wiederholte Druckerhöhungen werden damit vermieden. Zu beachten ist, dass dieses Erfordernis der genau einmaligen Druckerhöhung durch den Recyclekompressor nur den unreagierten Wasserstoff aus der ersten Reaktorstufe betrifft, welcher auch im Rückgewinnungsstrom enthalten ist. Sollte es also - wie regelmäßig der Fall ist - unreagierten Wasserstoff aus der ersten Reaktorstufe geben, welcher nicht in dem Rückgewinnungsstrom enthalten ist, so ist es nicht erforderlich, dass auch dieser unreagierte Wasserstoff außerhalb des Rückgewinnungsstroms genau eine Druckerhöhung durch den Recyclekompressor erfährt. Vielmehr ist dann sowohl eine mehrfache Druckerhöhung als auch überhaupt keine Druckerhöhung möglich.

Wie untenstehend noch beschrieben wird, kann diese erneute Zuführung des unreagierten Wasserstoffs zu der ersten Reaktorstufe indirekt derart erfolgen, dass der Wasserstoff als Teil einer Reihe weiterer Ströme der ersten Reaktorstufe zugeführt wird.

Grundsätzlich kann es sich bei der obigen Druckerhöhung des unreagierten Wasserstoffs um eine Druckerhöhung um einen beliebigen Betrag handeln. Bevorzugt ist, dass der unreagierte Wasserstoff vor der zumindest teilweisen Umwandlung in Methanol auf einen Druck erhöht wird, welcher höher als der Druck des H-Recyclestroms aus der Wasserstoffrückgewinnungsanordnung ist. Ebenso kann es sein, dass der unreagierte Wasserstoff vor der erneuten Zuführung zur ersten Reaktorstufe auf einen Druck erhöht wird, welcher höher als der Druck des Rückgewinnungsstroms bei Zuführung zu der Wasserstoffrückgewinnungsanordnung ist.

Die Druckerhöhung des unreagierten Wasserstoffs kann einerseits vor der Zuführung zur Wasserstoffrückgewinnungsanordnung erfolgen. So kann es sein, dass der Rückgewinnungsstrom insgesamt druckerhöht wird. Die Druckerhöhung des unreagierten Wasserstoffs kann aber auch nach Zuführung zur Wasserstoffrückgewinnungsanordnung erfolgen. Daher kann etwa die Druckerhöhung des unreagierten Wasserstoffs des H-Recyclestroms dadurch erfolgen, dass der H-Recyclestrom insgesamt druckerhöht wird.

Die Synthesegasreaktoranordnung, die Methanol-Reaktoranordnung, die Wärmerückgewinnungsvorrichtung, der Recyclekompressor und die Wasserstoffrückgewinnungsanordnung können von einer Anlage zur Synthese von Methanol umfasst sein.

Vorzugsweise beträgt der Erzeugungsdruck mehr als 60 bar oder mehr als 70 bar oder mehr als 80 bar. Der Erzeugungsdruck kann auch mehr als 90 bar und insbesondere mehr als 100 bar betragen.

Grundsätzlich kann der dem Recyclekompressor zugeführte Restgasstrom eine beliebige Zusammensetzung aufweisen, solange der Restgasstrom unreagierte Kohlenstoffoxide zu einem grundsätzlich beliebigen Anteil und den unreagierten Wasserstoff des Rückgewinnungsstroms umfasst. Bevorzugt ist jedoch, dass der dem Recyclekompressor zugeführte Restgasstrom einen molaren Wasserstoffanteil kleiner als 90 %, insbesondere kleiner als 85 % und weiter insbesondere kleiner als 80 % aufweist. Alternativ oder zusätzlich kann es sein, dass der dem Recyclekompressor zugeführte Restgasstrom einen molaren Wasserstoffanteil von größer als 50 %, insbesondere von größer als 60 % und weiter insbesondere von größer als 70 % aufweist. Dieser molare Wasserstoffanteil bezieht sich auf den gesamten molaren Wasserstoffanteil des Restgasstroms. Daher zählt nicht nur der Wasserstoff aus dem Rückgewinnungsstrom dazu, sondern auch sonstiger Wasserstoff in dem Restgasstrom.

Vorzugsweise umfasst die Methanol-Reaktoranordnung eine Methanol-Trennvorrichtung zum Gewinnen des unreagierten Restgases der ersten Reaktorstufe und eines Rohmethanolstroms der ersten Reaktorstufe. Grundsätzlich kann die Methanol-Trennvorrichtung auf beliebige Art und Weise funktionieren. Insbesondere kann es sein, dass die Methanol-Trennvorrichtung eine Kondensationsvorrichtung zum Gewinnen des unreagierten Restgases der ersten Reaktorstufe und des Rohmethanolstroms der ersten Reaktorstufe durch Kondensation umfasst.

Es kann sein, dass nur ein Teil des druckerhöhten Restgasstroms der Methanol-Reaktoranordnung zugeführt wird. Insbesondere ist es bevorzugt, dass ein Teil des druckerhöhten Restgasstroms abgezweigt und der Synthesegasreaktoranordnung zugeführt wird. Dabei kann insbesondere vorgesehen sein, dass der abgezweigte Teil des druckerhöhten Restgasstroms dem Energieträgerstrom zugeführt wird.

Wie bereits festgestellt kann es prinzipiell sein, dass die Methanol-Reaktoranordnung nur eine einzige Methanol-Reaktorstufe umfasst. Eine weitere bevorzugte Ausführungsform des Verfahrens ist dadurch gekennzeichnet, dass die Methanol-Reaktoranordnung eine Vielzahl von prozesstechnisch hintereinander geschalteten Reaktorstufen zur Methanolsynthese aufweist. Jede einzelne Reaktorstufe kann dabei einen oder mehrere Reaktoren aufweisen. Die Reaktoren einer Reaktorstufe können dabei insbesondere untereinander prozesstechnisch parallel angeordnet sein. Weiter kann es sein, dass durch die Methanol-Trennvorrichtung ein jeweiliges unreagiertes Restgas aus jeder der Vielzahl von Reaktorstufen gewonnen wird.

Dass die Reaktorstufen prozesstechnisch hintereinandergeschaltet sind bedeutet, dass Restgas aus einer Reaktorstufe - sofern es nicht die letzte Reaktorstufe in der Reihe der Reaktorstufen ist - direkt oder indirekt der jeweils danach geschalteten Reaktorstufe zugeführt wird. Grundsätzlich kann der obige Recyclekompressor bezüglich der Vielzahl von Reaktorstufen beliebig angeordnet sein. Eine Variante ist, dass der Recyclekompressor prozesstechnisch zwischen zwei Reaktorstufen angeordnet ist. Das bedeutet, dass dem Recyclekompressor zumindest ein Teil des unreagierten Restgases aus einer Reaktorstufe als Restgasstrom zugeführt wird und der druckerhöhte Restgasstrom dann der dieser Reaktorstufe nachgelagerten Reaktorstufe zugeführt wird.

Grundsätzlich kann der H-Recyclestrom beliebig geführt werden, solange mindestens ein Teil seines Wasserstoffs in Methanol umgewandelt wird. Gemäß einer weiteren bevorzugten Ausführungsform des Verfahrens ist diesbezüglich bevorzugt, dass der H-Recyclestrom dem unreagierten Restgas einer prozesstechnisch der ersten Reaktorstufe nachgelagerten Reaktorstufe zugeführt wird. Mit anderen Worten wird der unreagierte Wasserstoff des H-Recyclestroms nach der Zuführung gemeinsam mit zumindest einem Teil des unreagierten Restgases einer anderen Reaktorstufe als der ersten Reaktorstufe behandelt.

Bevorzugt ist, dass der H-Recyclestrom dem Recyclekompressor zur Druckerhöhung mit dem Restgasstrom zugeführt wird.

Gemäß einer bevorzugten Ausführungsform des Verfahrens ist vorgesehen, dass der Restgasstrom aus einer prozesstechnisch der ersten Reaktorstufe nachgelagerten Reaktorstufe gewonnen wird. Mit anderen Worten entstammt dann der dem Recyclekompressor zugeführte Restgasstrom nicht der ersten Reaktorstufe - also der Reaktorstufe, welcher der Synthesegasstrom unmittelbar zugeführt wird - sondern einer nachgelagerten Reaktorstufe. Weiter kann es sein, dass der Recyclekompressor den druckerhöhten Restgasstrom der ersten Reaktorstufe zuführt. Grundsätzlich kann der druckerhöhte Restgasstrom aber auch einer anderen Reaktorstufe der Vielzahl von Reaktorstufen zugeführt werden.

Eine weitere bevorzugte Ausführungsform des vorschlagsgemäßen Verfahrens ist dadurch gekennzeichnet, dass der Restgasstrom aus einer prozesstechnisch zuletztgelagerten Reaktorstufe der Vielzahl von Reaktorstufen gewonnen wird.

Grundsätzlich kann der Rückgewinnungsstrom an einer beliebigen Stelle und aus einem beliebigen Ursprung innerhalb der Methanol-Reaktoranordnung gewonnen werden. Erforderlich ist lediglich, dass er unreagierten Wasserstoff aus einem unreagierten Restgas der ersten Reaktorstufe enthält. Eine erste bevorzugte Variante sieht vor, dass der Rückgewinnungsstrom zumindest teilweise aus dem unreagierten Restgas der ersten Reaktorstufe abgezweigt wird. Es kann sein, dass der Rückgewinnungsstrom zumindest teilweise dem Recyclekompressor prozesstechnisch vorgelagert abgezweigt wird.

Es kann aber auch sein, dass der Rückgewinnungsstrom bereits eine Druckerhöhung durch den Recyclekompressor erfahren hat. Eine weitere bevorzugte Ausführungsform des Verfahrens ist daher dadurch gekennzeichnet, dass der Rückgewinnungsstrom der Wasserstoffrückgewinnungsanordnung mit einem Zuführungsdruck zugeführt wird, welcher höher ist als ein Restgasdruck, mit welchem der Restgasstrom aus der Methanol-Reaktoranordnung gewonnen wird. Eine bevorzugte Möglichkeit zur Erhöhung des Drucks des Rückgewinnungsstroms besteht darin, diesen zuvor durch den Recyclekompressor komprimieren zu lassen. Entsprechend ist es bevorzugt, dass der Rückgewinnungsstrom zumindest teilweise dem Recyclekompressor prozesstechnisch nachgelagert aus dem Restgasstrom abgezweigt wird.

Es kann aber auch sein, dass der Wasserstoffrückgewinnungsanordnung mehr als ein Strom zugeführt wird, aus dem Wasserstoff gewonnen wird. Gemäß einer bevorzugten Ausführungsform des Verfahrens ist so etwa vorgesehen, dass zumindest ein Teil des Synthesegasstroms für die Zuführung an eine Wassergas-Shift-Reaktionsvorrichtung abgezweigt wird. Es kann auch der Synthesegasstrom insgesamt der Wassergas-Shift-Reaktionsvorrichtung zugeführt werden. Ebenso ist es bevorzugt, dass ein weiterer Rückgewinnungsstrom zumindest teilweise aus der Wassergas-Shift-Reaktionsvorrichtung gewonnen und der Wasserstoffrückgewinnungsanordnung zum Gewinnen des H-Recyclestroms zugeführt wird. Mit anderen Worten wird zumindest ein Teil des Wasserstoffs des H-Recyclestroms aus diesem weiteren Rückgewinnungsstrom gewonnen. Diese Wassergas-Shift-Reaktionsvorrichtung kann von der Anlage zur Synthese von Methanol umfasst sein.

Insbesondere kann in der Wassergas-Shift-Reaktionsvorrichtung durch eine Wassergas-Shift-Reaktion zumindest ein Teil des Kohlenstoffoxids im Synthesegasstrom zu Kohlenstoffdioxid und Wasserstoff reagieren. Durch die Erhöhung des Wasserstoffanteils kann somit die Stöchiometrie für die Methanolsynthese verbessert werden.

Ebenso kann es sein, dass der H-Recyclestrom zunächst nicht der ersten Reaktorstufe zugeführt wird. Gemäß einer weiteren bevorzugten Ausführungsform des Verfahrens ist dann vorgesehen, dass der H-Recyclestrom dem Synthesegasstrom zugeführt wird. Das bedeutet speziell, dass der H-Recyclestrom dem Synthesegasstrom der Synthesegasreaktoranordnung prozesstechnisch nachgelagert zugeführt wird. Anders ausgedrückt wird der H-Recyclestrom dem Synthesegasstrom der ersten Reaktorstufe prozesstechnisch vorgelagert zugeführt. Durch die Zuführung des Synthesegasstroms zur ersten Reaktorstufe der Methanol-Reaktoranordnung wird der Wasserstoff des H-Recyclestroms im Ergebnis aber wieder der ersten Reaktorstufe zugeführt.

Die Synthesegasreaktoranordnung kann neben einem Reaktor zur Erzeugung des Synthesegases weitere Vorrichtungen aufweisen. So kann die Synthesegasreaktoranordnung eine jeweils dem Reaktor prozesstechnisch vorgelagerte Vorrichtung zur Entschwefelung des kohlenstoffhaltigen Energieträgerstroms, eine Sättigungsstufe zur Sättigung des kohlenstoffhaltigen Energieträgerstroms mit Wasser, einen Pre-Reformer zur Vorreformierung des kohlenstoffhaltigen Energieträgerstroms und/oder eine Vorrichtung zum Aufheizen des kohlenstoffhaltigen Energieträgerstroms aufweisen.

Grundsätzlich kann das Gewinnen des Synthesegasstroms aus dem Energieträgerstrom auf beliebige Art und Weise erfolgen. Bevorzugt ist, dass zum Gewinnen des Synthesegasstroms ein sauerstoffhaltiger Strom der Synthesegasreaktoranordnung zugeführt wird. Grundsätzlich kann der sauerstoffhaltige Strom neben dem Sauerstoff noch weitere Bestandteile aufweisen. So kann es sich bei dem sauerstoffhaltigen Strom auch um Umgebungsluft handeln.

Grundsätzlich kann der Synthesegasstrom etwa durch eine Dampfreformierung des kohlenstoffhaltigen Energieträgerstroms gewonnen werden. Eine weitere bevorzugte Ausführungsform des Verfahrens ist dadurch gekennzeichnet, dass in der Synthesegasreaktoranordnung der Synthesegasstrom durch eine autotherme Reformierung aus dem kohlenstoffhaltigen Energieträgerstrom gewonnen wird. Bei einer solchen autothermen Reformierung stellt eine katalytische partielle Oxidation die für die endothermen Reformierungsreaktionen erforderliche Wärme bereit. Gegenüber einer reinen Dampfreformierung bietet die autotherme Reformierung den Vorteil, dass der Synthesegasstrom mit einem höheren Druck bereitgestellt werden kann. Alternativ oder zusätzlich kann es sein, dass in der Synthesegasreaktoranordnung der Synthesegasstrom durch eine partielle Oxidation aus dem kohlenstoffhaltigen Energieträgerstrom gewonnen wird.

Grundsätzlich kann eine autotherme Reformierung auch mit Umgebungsluft betrieben werden. Bevorzugt ist jedoch, dass der sauerstoffhaltige Strom aus einer Luftzerlegungsvorrichtung zum Gewinnen eines Sauerstoffstroms aus einer Umgebungsluft gewonnen wird. Die Luftzerlegungsvorrichtung kann darüber hinaus auch zum Gewinnen eines Stickstoffstroms eingerichtet sein. Insbesondere kann es dann sein, dass der sauerstoffhaltige Strom im Wesentlichen aus Sauerstoff besteht. Auf diese Weise wird der Anteil an inerten Gasen bei der Methanolsynthese verringert, sodass verschiedene Vorrichtungen der Anlage kleiner dimensioniert werden können.

Gemäß einer bevorzugten Ausführungsform des Verfahrens ist vorgesehen, dass der H-Recyclestrom dem Energieträgerstrom zugeführt wird. Insbesondere kann es sein, dass der H-Recyclestrom dem Energieträgerstrom der Synthesegasreaktoranordnung prozesstechnisch vorgelagert zugeführt wird.

Neben dem H-Recyclestrom kann die Wasserstoffrückgewinnungsanordnung auch weitere Ströme ausgeben. Vorzugsweise gibt die Wasserstoffrückgewinnungsanordnung einen Purgestrom aus. Dieser kann insbesondere zur Verfeuerung abgeführt werden.

Grundsätzlich kann der H-Recyclestrom eine beliebige Zusammensetzung aufweisen, sofern er den unreagierten Wasserstoff enthält. Gemäß einer weiteren bevorzugten Ausführungsform des Verfahrens ist vorgesehen, dass der H-Recyclestrom einen höheren molaren Anteil an Wasserstoff als der Rückgewinnungsstrom aufweist. Mit anderen Worten ist in dem H-Recyclestrom der Wasserstoff gegenüber dem Rückgewinnungsstrom angereichert. Ebenso ist es bevorzugt, dass der H-Recyclestrom einen höheren molaren Anteil an Wasserstoff als der Purgestrom aufweist.

Grundsätzlich kann die Wasserstoffrückgewinnungsanordnung nach einem beliebigen Prinzip funktionieren, so etwa basierend auf einer Membran oder einer Kältevorrichtung. Eine bevorzugte Ausführungsform des Verfahrens ist dadurch gekennzeichnet, dass die Wasserstoffrückgewinnungsanordnung eine Druckwechsel-Adsorptionsvorrichtung (PSA) zum Gewinnen des H-Recyclestroms aus dem Rückgewinnungsstrom aufweist. Auf diese Weise kann eine hohe Rückgewinnung an Wasserstoff im H-Recyclestrom erreicht werden. Ebenso sind die Druckverluste bei einer solchen Druckwechsel-Adsorptionsvorrichtung noch vertretbar. Eine hohe Wasserstoffreinheit ist zwar vorliegend grundsätzlich nicht erforderlich, kann aber erreicht werden. Es kann also sein, dass der H-Recyclestrom im Wesentlichen aus Wasserstoff besteht.

Die vorschlagsgemäße Anlage dient der Synthese von Methanol. Sie weist eine Synthesegasreaktoranordnung zum Gewinnen eines Synthesegasstroms mit Wasserstoff und Kohlenstoffoxiden aus einem kohlenstoffhaltigen Energieträgerstrom, eine Methanol-Reaktoranordnung, welche eine erste Reaktorstufe aufweist, eine Wärmerückgewinnungsvorrichtung zum Rückgewinnen von Wärme aus dem Synthesegasstrom, eine Wasserstoffrückgewinnungsanordnung und einen Recyclekompressor auf.

Bei der vorschlagsgemäßen Anlage wird der Synthesegasstrom der ersten Reaktorstufe zur teilweisen Umwandlung in Methanol zugeführt und in der Synthesegasreaktoranordnung mit einem Erzeugungsdruck gewonnen, welcher höher ist als der Synthesedruck, mit welchem der Synthesegasstrom in der ersten Reaktorstufe teilweise in Methanol umgewandelt wird.

Weiter wird bei der vorschlagsgemäßen Anlage aus der Methanol-Reaktoranordnung ein Restgasstrom mit unreagierten Kohlenstoffoxiden gewonnen, welcher Restgasstrom dem Recyclekompressor zur Druckerhöhung des Restgasstroms zugeführt wird, wobei der druckerhöhte Restgasstrom der Methanol-Reaktoranordnung zur teilweisen Umwandlung in Methanol zugeführt wird, wobei vor Zuführung zu der ersten Reaktorstufe der Synthesegasstrom der Wärmerückgewinnungsvorrichtung zugeführt wird, wobei der Wasserstoffrückgewinnungsvorrichtung ein Rückgewinnungsstrom mit unreagiertem Wasserstoff aus einem unreagierten Restgas der ersten Reaktorstufe zum Gewinnen eines H-Recyclestroms mit dem unreagierten Wasserstoff des Rückgewinnungsstroms zugeführt wird, welcher unreagierte Wasserstoff des Rückgewinnungsstroms erneut der ersten Reaktorstufe zur zumindest teilweisen Umwandlung in Methanol zugeführt wird.

Die vorschlagsgemäße Anlage ist dadurch gekennzeichnet, dass der unreagierte Wasserstoff des Rückgewinnungsstroms von der ersten Reaktorstufe bis zur erneuten Zuführung zur ersten Reaktorstufe durch den Recyclekompressor mit den unreagierten Kohlenstoffoxiden genau einmal druckerhöht wird.

Merkmale, Vorteile und Eigenschaften der vorschlagsgemäßen Anlage entsprechen den Merkmalen, Vorteilen und Eigenschaften des vorschlagsgemäßen Verfahrens und umgekehrt.

Weitere Einzelheiten, Merkmale, Ziele und Vorteile der vorliegenden Erfindung werden nachfolgend anhand der nur Ausführungsbeispiele wiedergebenden Zeichnung erläutert. In der Zeichnung zeigt
- Fig. 1: schematisch das Fließbild einer Anlage zur Ausführung des vorschlagsgemäßen Verfahrens gemäß einem ersten Ausführungsbeispiel,
- Fig. 2: schematisch das Fließbild einer Anlage zur Ausführung des vorschlagsgemäßen Verfahrens gemäß einem zweiten Ausführungsbeispiel,
- Fig. 3: schematisch das Fließbild einer Anlage zur Ausführung des vorschlagsgemäßen Verfahrens gemäß einem dritten Ausführungsbeispiel,
- Fig. 4: schematisch das Fließbild einer Anlage zur Ausführung des vorschlagsgemäßen Verfahrens gemäß einem vierten Ausführungsbeispiel und
- Fig. 5: schematisch das Fließbild einer Anlage zur Ausführung des vorschlagsgemäßen Verfahrens gemäß einem fünften Ausführungsbeispiel.

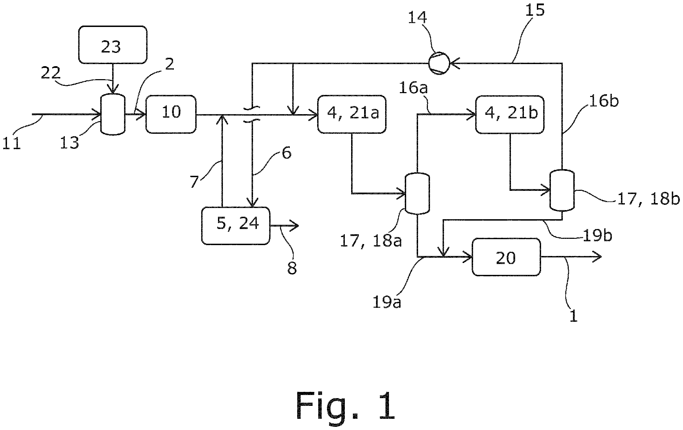

Die in der Fig. 1 gezeigte Anlage gemäß einem ersten Ausführungsbeispiel der vorschlagsgemäßen Anlage dient der Synthese von Methanol 1 und kann gemäß dem vorschlagsgemäßen Verfahren betrieben werden.

Ein im Wesentlichen aus Wasserstoff, Kohlenstoffmonoxid und Kohlenstoffdioxid bestehender Synthesegasstrom 2 wird aus einem durch Erdgas gebildeten und damit kohlenstoffhaltigen Energieträgerstrom 11 gewonnen, welcher einer Synthesegasreaktoranordnung 13 zugeführt wird. In der Synthesegasreaktoranordnung 13 findet eine autotherme Reformierung zum Gewinnen des Synthesegasstroms 2 statt. Für die autotherme Reformierung wird ein sauerstoffhaltiger Strom 22 zugeführt, welcher hier aus einer Luftzerlegungsvorrichtung 23 gewonnen wurde und im Wesentlichen aus Sauerstoff besteht. Die Luftzerlegungsvorrichtung 23 ist dabei zum Gewinnen eines Sauerstoffstroms - hier also des sauerstoffhaltigen Stroms 22 - aus der Umgebungsluft eingerichtet. Der Synthesegasstrom 2 wird mit einem Erzeugungsdruck von im Wesentlichen 80 bar gewonnen.

Der Synthesegasstrom 2 wird zunächst einer Wärmerückgewinnungsvorrichtung zugeführt 10, in welcher der Synthesegasstrom 2 abgekühlt wird und auf diese Weise ein Teil der bei der autothermen Reformierung erzeugten Wärme zurückgewonnen wird. Anschließend wird der Synthesegasstrom 2 der ersten Reaktorstufe 21a einer Methanol-Reaktoranordnung 4 zugeführt, in welcher ersten Reaktorstufe 21a eine Methanolsynthese stattfindet und zumindest ein Teil des Synthesegasstroms 2 in Methanol 1 umgewandelt wird. Die Methanolsynthese findet bei einem Synthesedruck von über 70 bar statt. Ein Synthesegaskompressor zur Druckerhöhung des Synthesegasstroms 2 ist daher entbehrlich.

Die Anlage weist eine als Druckwechsel-Adsorptionsanlage 24 - welche auch als PSA bezeichnet werden kann - ausgebildete Wasserstoffrückgewinnungsanordnung 5 auf, welche aus einem Rückgewinnungsstrom 6 einen H-Recyclestrom 7 gewinnt, welcher H-Recyclestrom 7 im Wesentlichen aus Wasserstoff besteht. Ebenso wird das verbleibende Gas von der Wasserstoffrückgewinnungsanordnung 5 als Purgestrom 8 ausgegeben und anschließend in einer - hier nicht dargestellten - befeuerten Heizvorrichtung der Anlage verfeuert. Der H-Recyclestrom 7 wird dem Synthesegasstrom 2 zugeführt.

Wie in der Fig. 1 zu erkennen ist, weist die Anlage des ersten Ausführungsbeispiels ebenso einen Recyclekompressor 14 auf, welcher einen Restgasstrom 15 komprimiert. Der Restgasstrom 15 weist unreagiertes Restgas 16b auf, welches seinerseits im Wesentlichen diejenigen Bestandteile des Synthesegases aufweist, welche in der Methanol-Reaktoranordnung 4 nicht in Methanol 1 umgewandelt wurden. Entsprechend weist der Restgasstrom 15 insbesondere unreagierte Kohlenstoffoxide auf. Der somit druckerhöhte Restgasstrom 15 wird zu einem ersten Teil erneut der Methanol-Reaktoranordnung 4 zugeführt.

Das unreagierte Restgas 16a, b wird aus einer Methanol-Trennvorrichtung 17 der Methanol-Reaktoranordnung 4 gewonnen, welche hier zwei Kondensationsvorrichtungen 18a, b umfasst. Durch Kondensation wird in diesen jeweils das unreagierte Restgas 16a, b einerseits und ein jeweiliger Rohmethanolstrom 19a, b andererseits gewonnen. Die Rohmethanolströme 19a, b werden dann einer Destillation 20 der Anlage zugeführt, sodass das Methanol 1 aus den Rohmethanolströmen 19a, b gewonnen werden kann.

Bei der Anlage des Ausführungsbeispiels der Fig. 1 weist die Methanol-Reaktoranordnung 4 zwei prozesstechnisch hintereinander geschaltete Reaktorstufen 21a, b zur Methanolsynthese auf. Bei diesem Ausführungsbeispiel weist die erste Reaktorstufe 21a zwei zueinander parallel angeordnete isotherme Reaktoren und die zweite Reaktorstufe 21b einen einzelnen isothermen Reaktor auf. Jeder der beiden Kondensationsvorrichtungen 18a, b wird dabei der Produktstrom aus jeweils einer Reaktorstufe 21a, b zugeführt. Dabei wird diejenige Reaktorstufe 21a, welcher der Synthesegasstrom 2 direkt zugeführt wird, als erste Reaktorstufe 21a bezeichnet. Die Reaktorstufe 21b ist dieser dann in dem Sinne prozesstechnisch nachgelagert, dass ihr das unreagierte Restgas 16a aus der ersten Reaktorstufe 21a zur Umwandlung in Methanol 1 zugeführt wird.

Bei diesem Ausführungsbeispiel der Fig. 1 wird der Rückgewinnungsstrom 6 aus dem durch den Recyclekompressor druckerhöhten Restgasstrom 15 abgezweigt. Dieser dem Recyclekompressor 14 zugeführte Restgasstrom 15 wird nicht aus dem unreagierten Restgas 16a der ersten Reaktorstufe 21a gewonnen, sondern aus dem unreagierten Restgas 16b der der ersten Reaktorstufe 21a prozesstechnisch nachgelagerten und damit zweiten Reaktorstufe 21b.

Gleichwohl weist dieser Restgasstrom 15 neben den bereits genannten unreagierten Kohlenstoffoxiden auch unreagierten Wasserstoff aus der ersten Reaktorstufe 21a auf. Den jedweder unreagierte Wasserstoff aus dem Restgas 16a der ersten Reaktorstufe 21a wird der zweiten Reaktorstufe 21b zugeführt. Da auch in der zweiten Reaktorstufe 21b keine vollständige Reaktion des Wasserstoffs stattfindet, weist das unreagierte Restgas 16b der zweiten Reaktorstufe 21b auch unreagierten Wasserstoff aus der ersten Reaktorstufe 21a auf.

Da der Rückgewinnungsstrom 6 aus dem druckerhöhten Restgasstrom 15 abgezweigt wurde, weist auch der H-Recyclestrom 7 unreagierten Wasserstoff aus dem Restgas 16a der ersten Reaktorstufe 21a auf. Speziell wird ein zweiter Teil des druckerhöhten Restgasstroms 15 als Rückgewinnungsstrom 6 abgezweigt. Dadurch, dass der H-Recyclestrom 7 dem Synthesegasstrom 2 zugeführt wird, wird der unreagierte Wasserstoff aus dem Restgas 16a der ersten Reaktorstufe 21a im Rückgewinnungsstrom 6 dieser ersten Reaktorstufe 21 wieder für die Umwandlung in Methanol zugeführt. Zwischen dem Verlassen der ersten Reaktorstufe 21a und der neuerlichen Zufuhr zu der ersten Reaktorstufe 21a hat der unreagierte Wasserstoff des Rückgewinnungsstroms 6 jedoch als Bestandteil des Restgasstroms 15 eine Druckerhöhung durch den Recyclekompressor 14 erfahren, und zwar genau einmal und gemeinsam mit den unreagierten Kohlenstoffoxiden im Restgasstrom 15. Der durch den Recyclekompressor 14 komprimierte Restgasstrom 15 wird dann wiederum zu dem bereits erwähnten ersten Teil direkt der ersten Reaktorstufe 21a zugeführt.

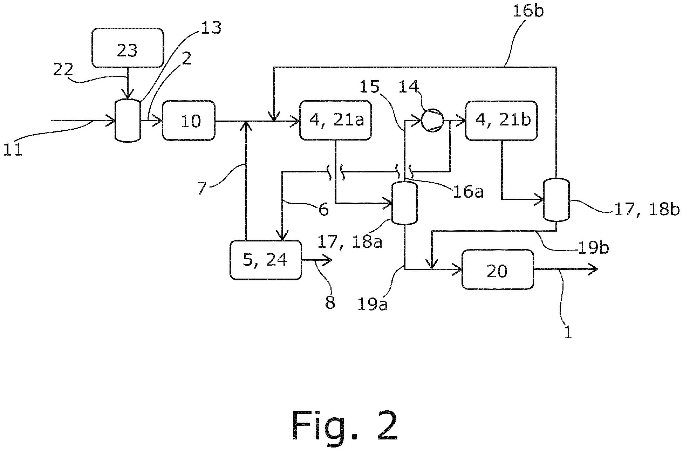

Das zweite Ausführungsbeispiel der vorschlagsgemäßen Anlage, dargestellt in der Fig. 2, unterscheidet sich von dem Ausführungsbeispiel der Fig. 1 darin, dass der Recyclekompressor 14 prozesstechnisch zwischen der ersten Reaktorstufe 21a und der ihr nachgelagerten Reaktorstufe 21b angeordnet ist. Folglich wird der dem Recyclekompressor 14 zugeführte Restgasstrom 15 aus dem unreagierten Restgas 16a der ersten Reaktorstufe 21a gewonnen. Der durch den Recyclekompressor 14 komprimierte Restgasstrom 15 mit den unreagierten Kohlenstoffoxiden wird der der ersten Reaktorstufe 21a nachgelagerten Reaktorstufe 21b zugeführt. Das unreagierte Restgas 16b aus dieser Reaktorstufe 21b wird ohne weitere Komprimierung zurück zur ersten Reaktorstufe 21a geführt. Der Rückgewinnungsstrom 6 wird - anders als bei dem ersten Ausführungsbeispiel - aus dem unreagierten Restgas 16a der ersten Reaktorstufe 21a gewonnen, wobei ebenfalls in Übereinstimmung mit dem ersten Ausführungsbeispiel das Abzweigen des Rückgewinnungsstroms 6 dem Recyclekompressor 14 prozesstechnisch nachgelagert erfolgt. Folglich findet auch bei dem zweiten Ausführungsbeispiel eine Druckerhöhung des unreagierten Wasserstoffs aus dem Restgas 16a der ersten Reaktorstufe 21a im Rückgewinnungsstrom 6 mit den unreagierten Kohlenstoffoxiden durch den Recyclekompressor 14 genau einmal statt, bevor dieser unreagierte Wasserstoff wieder der ersten Reaktorstufe 21a zugeführt wird.

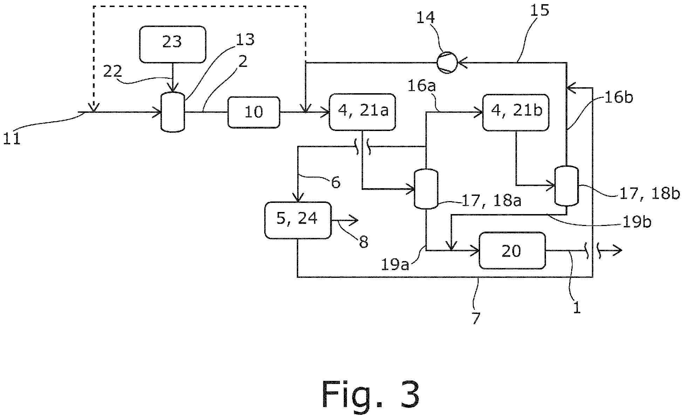

Bei dem dritten Ausführungsbeispiel der Fig. 3 wird der Rückgewinnungsstrom 6 ähnlich wie bei dem zweiten Ausführungsbeispiel aus dem Restgas 16a der ersten Reaktorstufe 21a gewonnen. Im Gegensatz zum zweiten Ausführungsbeispiel ist jedoch kein Recyclekompressor 14 zwischen der ersten Reaktorstufe 21a und der zweiten Reaktorstufe 21b angeordnet. Vielmehr ist der Recyclekompressor 14 wie bei dem ersten Ausführungsbeispiel der zweiten Reaktorstufe 21b prozesstechnisch nachgelagert angeordnet.

Anders als sowohl dem ersten Ausführungsbeispiel und dem zweiten Ausführungsbeispiel wird bei dem dritten Ausführungsbeispiel der H-Recyclestrom 7 dem Restgas 16b der der ersten Reaktorstufe 21a nachgelagerten zweiten Reaktorstufe 21b zugeführt. Speziell erfolgt diese Zuführung vor der Druckerhöhung durch den Recyclekompressor 14. Der Wasserstoff in dem H-Recyclestrom 7 entsprechend dem unreagierten Wasserstoff aus dem Restgas 16a der ersten Reaktorstufe 21a im Rückgewinnungsstrom 6 erhält auf diese Weise mit dem sonstigen unreagierten Restgas 16b der zweiten Reaktorstufe 21b und insbesondere mit unreagierten Kohlenstoffoxiden eine Druckerhöhung durch den Recyclekompressor 14. Diese Druckerhöhung erfolgt also genau einmal vor der erneuten Zuführung dieses unreagierten Wasserstoffes zu der ersten Reaktorstufe 21a, was die mangels Synthesegaskompressors fehlende Druckerhöhung kompensiert.

Zusätzlich ist bei dem dritten Ausführungsbeispiel vorgesehen, dass ein Teil des druckerhöhten Restgasstroms 15 abgezweigt und dem Energieträgerstrom 11 zugeführt wird. Es ist aber auch möglich, auf dieses Abzweigen eines Teils des druckerhöhten Restgasstroms 15 zu verzichten.

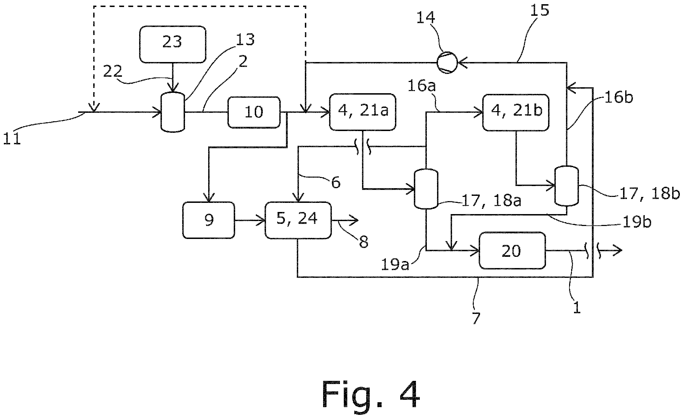

Die Anlage gemäß dem vierten Ausführungsbeispiel der Fig. 4 entspricht dem dritten Ausführungsbeispiel der Fig. 3. Sie weist jedoch eine Wassergas-Shift-Reaktionsvorrichtung 9 auf, welcher ein Teil des Synthesegasstroms 2 nach Zuführung zu der Wärmerückgewinnungsvorrichtung 10 zugeführt wird. Die in der Wassergas-Shift-Reaktionsvorrichtung 9 ablaufende Wassergas-Shift-Reaktion führt zu einer Erhöhung des Wasserstoffanteils in dem abgezweigten Teil des Synthesegasstroms 2. Der auf diese Weise abgezweigte und der Wassergas-Shift-Reaktion unterzogene Teil des Synthesegasstrom 2 aus der Wassergas-Shift-Reaktionsvorrichtung 9 bildet hier einen weiteren Rückgewinnungsstrom, welcher zusammen mit der Rückgewinnungsstrom 6 der Wasserstoffrückgewinnungsanordnung 5 zugeführt wird. Ebenso wie bei dem Ausführungsbeispiel der Fig. 3 wird der H-Recyclestrom 7 dem Restgas 16b der der ersten Reaktorstufe 21a nachgelagerten zweiten Reaktorstufe 21b zugeführt, sodass also auch bei diesem Ausführungsbeispiel eine einmalige Druckerhöhung durch den Recyclekompressor 14 mit den unreagierten Kohlenstoffoxiden erfolgt.

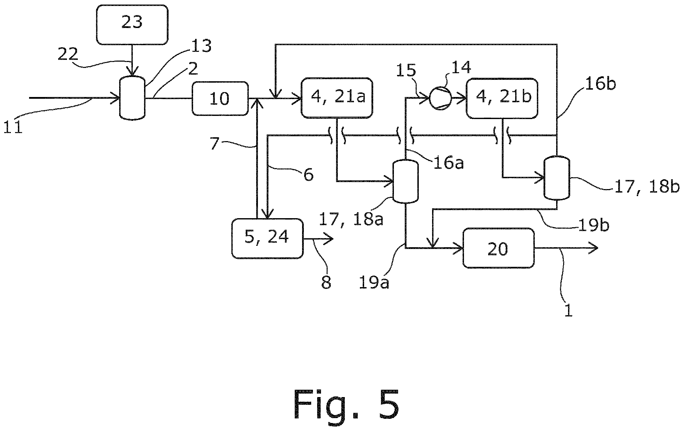

Das fünfte Ausführungsbeispiel der Fig. 5 sieht eine Anordnung des Recyclekompressors 14 zwischen den Reaktorstufen 21a, b der Methanol-Reaktoranordnung 4 vor wie bei dem zweiten Ausführungsbeispiel, auf welchem das fünfte Ausführungsbeispiel auch basiert. Im Unterschied zum zweiten Ausführungsbeispiel wird der Rückgewinnungsstrom 6 aus dem Restgas 16b der zweiten Reaktorstufe 21b gewonnen. Der Wasserstoff in diesem Rückgewinnungsstrom 6 hat dabei eine Druckerhöhung durch den Recyclekompressor erfahren, und zwar speziell noch vor Zuführung zu der zweiten Reaktorstufe 21b.

## Patentansprüche

1. Verfahren zur Synthese von Methanol (1), wobei ein kohlenstoffhaltiger Energieträgerstrom (11) einer Synthesegasreaktoranordnung (13) zum Gewinnen eines Synthesegasstroms (2) mit Wasserstoff und Kohlenstoffoxiden zugeführt wird, wobei der Synthesegasstrom (2) einer ersten Reaktorstufe (21a) einer Methanol-Reaktoranordnung (4) zur teilweisen Umwandlung in Methanol (1) zugeführt wird, wobei der Synthesegasstrom (2) in der Synthesegasreaktoranordnung (13) mit einem Erzeugungsdruck gewonnen wird, welcher höher ist als der Synthesedruck, mit welchem der Synthesegasstrom (2) in der ersten Reaktorstufe (21a) teilweise in Methanol (1) umgewandelt wird, wobei aus der Methanol-Reaktoranordnung (4) ein Restgasstrom (15) mit unreagierten Kohlenstoffoxiden gewonnen wird, welcher Restgasstrom (15) einem Recyclekompressor (14) zur Druckerhöhung des Restgasstroms (15) zugeführt wird, wobei der druckerhöhte Restgasstrom (15) der Methanol-Reaktoranordnung (4) zur teilweisen Umwandlung in Methanol (1) zugeführt wird, wobei vor Zuführung zu der ersten Reaktorstufe (21a) der Synthesegasstrom (2) einer Wärmerückgewinnungsvorrichtung (10) zum Rückgewinnen von Wärme aus dem Synthesegasstrom (2) zugeführt wird, wobei ein Rückgewinnungsstrom (6) mit unreagiertem Wasserstoff aus einem unreagierten Restgas (16a) der ersten Reaktorstufe (21a) einer Wasserstoffrückgewinnungsanordnung (5) zum Gewinnen eines H-Recyclestroms (7) mit dem unreagierten Wasserstoff des Rückgewinnungsstroms (6) zugeführt wird, welcher unreagierte Wasserstoff des Rückgewinnungsstroms (6) erneut der ersten Reaktorstufe (21a) zur zumindest teilweisen Umwandlung in Methanol (1) zugeführt wird, **dadurch gekennzeichnet, dass** der unreagierte Wasserstoff des Rückgewinnungsstroms (6) von der ersten Reaktorstufe (21a) bis zur erneuten Zuführung zur ersten Reaktorstufe (21a) durch den Recyclekompressor (14) mit den unreagierten Kohlenstoffoxiden genau einmal druckerhöht wird.

2. Verfahren nach Anspruch 1, **dadurch gekennzeichnet, dass** die Methanol-Reaktoranordnung (4) eine Methanol-Trennvorrichtung (17) zum Gewinnen des unreagierten Restgases (16a) der ersten Reaktorstufe (21a) und eines Rohmethanolstroms (19a) der ersten Reaktorstufe (21a) umfasst, insbesondere, dass die Methanol-Trennvorrichtung (17) eine Kondensationsvorrichtung (18a) zum Gewinnen des unreagierten Restgases (16a) der ersten Reaktorstufe (21a) und des Rohmethanolstroms (19a) der ersten Reaktorstufe (21a) durch Kondensation umfasst.

3. Verfahren nach Anspruch 2, **dadurch gekennzeichnet, dass** ein Teil des druckerhöhten Restgasstroms (15) abgezweigt und der Synthesegasreaktoranordnung (13) zugeführt wird, vorzugsweise dass der abgezweigte Teil des druckerhöhten Restgasstroms (15) dem Energieträgerstrom (11) zugeführt wird.

4. Verfahren nach einem der Ansprüche 1 bis 3, **dadurch gekennzeichnet, dass** die Methanol-Reaktoranordnung (4) eine Vielzahl von prozesstechnisch hintereinander geschalteten Reaktorstufen (21a, b) zur Methanolsynthese aufweist, vorzugsweise, dass der Recyclekompressor (14) prozesstechnisch zwischen zwei Reaktorstufen (21a, b) angeordnet ist, insbesondere, dass durch die Methanol-Trennvorrichtung (17) ein jeweiliges unreagiertes Restgas (16a, b) aus jeder der Vielzahl von Reaktorstufen (21a, b) gewonnen wird.

5. Verfahren nach Anspruch 4, **dadurch gekennzeichnet, dass** der H-Recyclestrom (7) dem unreagierten Restgas (16b) einer prozesstechnisch der ersten Reaktorstufe (21a) nachgelagerten Reaktorstufe (21b) zugeführt wird, vorzugsweise, dass der H-Recyclestrom (7) dem Recyclekompressor (14) zur Druckerhöhung gemeinsam mit dem Restgasstrom (15) zugeführt wird.

6. Verfahren nach Anspruch 4 oder 5, **dadurch gekennzeichnet, dass** der Restgasstrom (15) aus einer prozesstechnisch der ersten Reaktorstufe (21a) nachgelagerten Reaktorstufe (21b) gewonnen wird, insbesondere, dass der Recyclekompressor (14) den druckerhöhten Restgasstrom (15) der ersten Reaktorstufe (21a) zuführt.

7. Verfahren nach Anspruch 6, **dadurch gekennzeichnet,, dass** der Restgasstrom (15) aus einer prozesstechnisch zuletztgelagerten Reaktorstufe (21b) der Vielzahl von Reaktorstufen (21a, b) gewonnen wird.

8. Verfahren nach einem der Ansprüche 1 bis 7, **dadurch gekennzeichnet,, dass** der Rückgewinnungsstrom (6) zumindest teilweise aus dem unreagierten Restgas (16a) der ersten Reaktorstufe (21a) abgezweigt wird, weiter vorzugsweise, dass der Rückgewinnungsstrom (6) zumindest teilweise dem Recyclekompressor (14) prozesstechnisch vorgelagert abgezweigt wird.

9. Verfahren nach Anspruch 8, **dadurch gekennzeichnet, dass** der Rückgewinnungsstrom (6) der Wasserstoffrückgewinnungsanordnung (5) mit einem Zuführungsdruck zugeführt wird, welcher höher ist als ein Restgasdruck, mit welchem der Restgasstrom (15) aus der Methanol-Reaktoranordnung gewonnen wird, vorzugsweise, dass der Rückgewinnungsstrom (6) zumindest teilweise dem Recyclekompressor (14) prozesstechnisch nachgelagert aus dem Restgasstrom (15) abgezweigt wird.

10. Verfahren nach einem der Ansprüche 1 bis 9, **dadurch gekennzeichnet, dass** der H-Recyclestrom (7) dem Synthesegasstrom (2) zugeführt wird.

11. Verfahren nach einem der Ansprüche 1 bis 10, **dadurch gekennzeichnet, dass** zum Gewinnen des Synthesegasstroms (2) ein sauerstoffhaltiger Strom (22) der Synthesegasreaktoranordnung (13) zugeführt wird, insbesondere, dass in der Synthesegasreaktoranordnung (13) der Synthesegasstrom (2) durch eine autotherme Reformierung oder eine partielle Oxidation aus dem kohlenstoffhaltigen Energieträgerstrom (11) gewonnen wird, weiter vorzugsweise, dass der sauerstoffhaltige Strom (22) aus einer Luftzerlegungsvorrichtung (23) zum Gewinnen eines Sauerstoffstroms aus einer Umgebungsluft gewonnen wird, weiter, insbesondere, dass der sauerstoffhaltige Strom (22) im Wesentlichen aus Sauerstoff besteht.

12. Verfahren nach einem der Ansprüche 1 bis 11, **dadurch gekennzeichnet, dass** der H-Recyclestrom (7) dem Energieträgerstrom (11), insbesondere der Synthesegasreaktoranordnung (13) prozesstechnisch vorgelagert, zugeführt wird, vorzugsweise, dass die Wasserstoffrückgewinnungsanordnung (5) einen Purgestrom (8) ausgibt, welcher weiter insbesondere zur Verfeuerung abgeführt wird.

13. Verfahren nach einem der Ansprüche 1 bis 12, **dadurch gekennzeichnet, dass** der H-Recyclestrom (7) einen höheren molaren Anteil an Wasserstoff als der Rückgewinnungsstrom (6) aufweist, vorzugsweise, dass der H-Recyclestrom (7) einen höheren molaren Anteil an Wasserstoff als der Purgestrom (8) aufweist.

14. Verfahren nach einem der Ansprüche 1 bis 13, **dadurch gekennzeichnet, dass** die Wasserstoffrückgewinnungsanordnung (5) eine Druckwechsel-Adsorptionsvorrichtung (24) zum Gewinnen des H-Recyclestroms (7) aus dem Rückgewinnungsstrom (6) aufweist, weiter vorzugsweise, dass der H-Recyclestrom (7) im Wesentlichen aus Wasserstoff besteht.

15. Anlage zur Synthese von Methanol (1) mit einer Synthesegasreaktoranordnung (13) zum Gewinnen eines Synthesegasstroms (2) mit Wasserstoff und Kohlenstoffoxiden aus einem kohlenstoffhaltigen Energieträgerstrom (11), mit einer Methanol-Reaktoranordnung (4), welche eine erste Reaktorstufe (21a) aufweist, mit einer Wärmerückgewinnungsvorrichtung (10) zum Rückgewinnen von Wärme aus dem Synthesegasstrom (2), mit einer Wasserstoffrückgewinnungsanordnung (5) und mit einem Recyclekompressor (14), wobei der Synthesegasstrom (2) der ersten Reaktorstufe (21a) zur teilweisen Umwandlung in Methanol (1) zugeführt wird und in der Synthesegasreaktoranordnung (13) mit einem Erzeugungsdruck gewonnen wird, welcher höher ist als der Synthesedruck, mit welchem der Synthesegasstrom (2) in der ersten Reaktorstufe (21a) teilweise in Methanol (1) umgewandelt wird, wobei aus der Methanol-Reaktoranordnung (4) ein Restgasstrom (15) mit unreagierten Kohlenstoffoxiden gewonnen wird, welcher Restgasstrom (15) dem Recyclekompressor (14) zur Druckerhöhung des Restgasstroms (15) zugeführt wird, wobei der druckerhöhte Restgasstrom (15) der Methanol-Reaktoranordnung (4) zur teilweisen Umwandlung in Methanol (1) zugeführt wird, wobei vor Zuführung zu der ersten Reaktorstufe (21a) der Synthesegasstrom (2) der Wärmerückgewinnungsvorrichtung (10) zugeführt wird, wobei der Wasserstoffrückgewinnungsvorrichtung (5) ein Rückgewinnungsstrom (6) mit unreagiertem Wasserstoff aus einem unreagierten Restgas (16a) der ersten Reaktorstufe (21a) zum Gewinnen eines H-Recyclestroms (7) mit dem unreagierten Wasserstoff des Rückgewinnungsstroms (6) zugeführt wird, welcher unreagierte Wasserstoff des Rückgewinnungsstroms (6) erneut der ersten Reaktorstufe (21a) zur zumindest teilweisen Umwandlung in Methanol (1) zugeführt wird, **dadurch gekennzeichnet, dass** der unreagierte Wasserstoff des Rückgewinnungsstroms (6) von der ersten Reaktorstufe (21a) bis zur erneuten Zuführung zur ersten Reaktorstufe (21a) durch den Recyclekompressor (14) mit den unreagierten Kohlenstoffoxiden genau einmal druckerhöht wird.
